# EUROPEAN PATENT SPECIFICATION

(11) **EP 3 755 433 B1**
(45) Date of publication and mention of the grant of the patent: **23.08.2023**
(21) Application number: 19710165.2
(22) Date of filing: 22.02.2019
(51) Int. Cl.: A61K 31/728, A61K 31/355, A61K 31/205, A61K 31/19, A61K 31/197, A61K 33/30, A61K 33/38, A61K 35/20, A61K 36/886, A61K 36/28, A61K 9/00, A61K 47/44, A61P 15/02

(54) **COMPOSITION BASED ON A MIXTURE OF BIOLOGICAL FACTORS ISOLATED FROM COLOSTRUM AND OZONIZED VEGETABLE OILS FOR USE IN THE TREATMENT OF VAGINITIS AND VAGINOSIS**
ZUBEREITUNG ENHALTEND EIN GEMISCH AUS BIOLOGISCHEN FAKTOREN ISOLIERT AUS KOLOSTRUM UND OZONISIERTE VEGETARISCHE ÖLE ZUR BEHANDLUNG VON VAGINITIS UND VAGINOSIS
COMPOSITION À BASE D'UN MÉLANGE DE FACTEURS BIOLOGIQUES ISOLÉS À PARTIR DU COLOSTRUM ET D'HUILES VEGETALES OZONISEES POUR SON UTILISATION DANS LE TRAITEMENT DE LA VAGINITE ET DE LA VAGINOSIS

(30) Priority: 23.02.2018 IT 201800002990
(43) Date of publication of application: 30.12.2020
(73) Proprietor: Biomedical Research S.r.l., 95128 Catania (IT)
(72) Inventor: ZARBO, Giuseppa, 95128 Catania (IT)
(74) Representative: Croce, Valeria
(86) International application number: PCT/IB2019/051457
(87) International publication number: WO 2019/162895

(56) References cited:
- EP-A1- 1 273 295
- US-A1- 2014 322 329

## Description

The present invention relates to vaginal topical compositions, for use in the treatment of vaginitis and vaginosis.

### Technical field of the invention

For many years, the vaginal ecosystem has been considered a static and homogeneous entity consisting essentially of the Döderlein's bacillus (or *Lactobacillus*), an anaerobic *Gram* positive bacterium, and any variations with respect to this typical condition were considered pathological. Later, however, it was realized that in this ecological niche there is a set of microorganisms in dynamic equilibrium with the environment that hosts it and whose composition undergoes frequent and significant changes in the course of a woman's life, mainly affected by the physiological variations of the hormonal structure, in particular the circulating level of oestrogens, and other no less important factors such as: sexual activity, pregnancy, sanitary conditions, systemic diseases, pharmacological treatments (antibiotic or immunosuppressive therapies), radiation therapy, trauma.

Upon reaching sexual maturity, with the haematic reappearance of estrogens and the subsequent structural physiological changes of the vaginal mucosa (epithelial thickening, cellular pyknosis and glycogen synthesis), the woman acquires the typical microflora of the fertile age dominated by lactobacilli (95%); the most common species are: *L. iners, L. crispatus, L. gasseri, L. jenesenii,* followed by *L. acidophilus, L. fermentum, L. plantarum, L. brevis, L. casei, L. vaginalis, L. delbrueckii, L. salivarius, L. reuteri* and *L. rhamnosus.* Together with the local immune system, these microorganisms play a key role in the defense of the host, both through the formation of a "biofilm" that hinders the adhesion of pathogens, and due to the competition for metabolites, and to the production of different substances such as hydrogen peroxide, bacteriocins and lactic acid. The latter metabolite makes the endoluminal pH acidic and this is the most important factor limiting vaginal colonization by exogenous microorganisms and the growth of potentially pathogenic commensal bacteria. Lactic acid, whose presence is closely related to vaginal trophism and therefore indirectly to estrogens, derives mainly from the anaerobic metabolism of glucose by lactobacilli.

Another important defense mechanism is represented by hydrogen peroxide produced by some lactobacilli strains, mainly *L. crispatus* and *L. jenseii,* a toxic substance for a large number of catalase-peroxidase-free bacterial cells including *Gardnerella vaginalis, Escherichia coli* and *Staphylococcus aureus.* Furthermore, the combined action of hydrogen peroxide and uterine peroxidase (produced by the cervix and endometrium) limits bacterial growth by the activation of the polymorphonuclear cells that exert their bactericidal action in the intercellular epithelial spaces.

The vaginal ecosystem under physiological conditions therefore represents a dynamic balance of microbial flora modulated by the hormonal structure, the pH and the immune response, factors that are closely interdependent. Vaginal wall cells play an important role in maintaining this balance, in particular the superficial and intermediate layers of the epithelium, whose proliferation and maturation is hormone-related.

An important role is also exercised by the fluid, which, under physiological conditions, moistens the vaginal mucosa, visible to the inspection as a clear secretion consisting of plasma transudate, cervical mucus and vestibular gland secretions. This fluid in fact contains antimicrobial substances such as antibodies (IgG and IgA), cytokines and antibacterial factors, which give it a protective interface function and hinders microbial colonization by mechanical action, through its viscosity and elution properties.

When this delicate balance is lacking, various disorders of different entities arise that can be classified as 1) atrophic vaginitis 2) infectious vaginitis (*Candida* spp., *Trichomonas vaginalis, Chlamydia trachomatis, Escherichia coli, Herpes genitalis,* etc.); 3) bacterial vaginosis (*Gardnerella vaginalis,* etc.); 4) irritating/allergic vaginitis due to detergents, soaps, panty liners, lubricants, non-breathable garments; 5) Sjögren's syndrome. The diagnosis is based on the careful inspection of the external genitals in the search for signs of inflammation (rubor, tumor, calor, dolor) or signs of vulvo-vaginal atrophy; in particular, these are signs of involution, at the vulvar level: reduced elasticity and skin turgidity, thinning of pubic hair, dryness and reduction of the labia. The atrophic vaginal epithelium appears pale, glossy, thin and fragile. Once the vaginal pH alkalinization has been verified, the culture tests and the pap smear can help to detect evidence of *Candida, Trichomonas* or bacterial vaginosis; assessment of serum estrogen levels may help in early menopause.

Symptoms consist of pain, burning and itching, particularly during intercourse and/or urination; the disorders tend, then, to last over time, especially if there is a lack of vaginal and vulvar lubrication. Normally, the treatment is based not only on targeted antibiotic therapy and on precise hygiene-behavioral rules, but also on the use of topical estrogens for preventive purposes and, if necessary, on hormone replacement therapy: there are numerous hormone-based drugs proposed to cure these diseases, unfortunately for all these there is a suspicion of an increased incidence of mammary neoplasia.

The need for alternative therapeutic approaches stems from the frequent confirmation in clinical practice of relapses, which entail for the patient an important psychophysical discomfort, significantly altering the quality of life with significant health costs. The failure of antibiotic therapy, commonly used in the treatment of diseases of infectious origin, is linked to two main factors, such as: the development by pathogenic microorganisms of resistance to chemotherapeutics, favored in recent years by the wide spread of over-the-counter drugs more often advertised on women's blogs and forums who promote the improper use thereof as self-medication, and the negative impact of antibiotics on vaginal microflora, thus complicating the regeneration of lactobacilli which constitute the main line of defense of the vaginal ecosystem in women of child-bearing age. In fact, altered flora facilitates both colonization by exogenous micro-organisms and the virulence of potentially pathogenic commensal bacteria normally present at low concentrations.

The international patent application WO 2012/076409 describes preparations based on colostrum for the treatment of vaginal dryness, but does not describe the use of such formulations also for the treatment of vaginitis and vaginosis.

There is therefore the need to have preparations based on natural substances that do not alter the vaginal ecosystem and help restore and maintain the balance by strengthening its defenses, which promote the reconstruction and maintenance of the functional integrity of the vaginal mucosa (atrophic or damaged by bacterial and viral agents, detergents, soaps, panty liners, lubricants, non-breathable garments, pharmacological treatments, etc.) and which counteract the multiplication of pathogenic bacteria and viruses in vaginitis and vaginosis.

### Summary of the invention

The inventors of the present patent application have surprisingly found that a formulation according to the appended claims is able to solve the problems of the compositions known in the art.

### Object of the invention

In a first object, the present invention relates to a vaginal topical composition comprising a mixture isolated from colostrum, rich in biological factors, and ozonized vegetable oils.

In a preferred aspect, the composition may comprise further compounds having specific properties.

According to an aspect of the invention, the described compositions are in the form of gels, vaginal suppositories, douching or other suitable formulations.

In a second object, the present invention relates to the described composition for use in medicine.

In a preferred aspect, the composition is for use in the treatment of vaginal dryness, vaginitis and vaginosis.

### Brief description of the figures

Figure 1 shows the classification indexes of vaginal health;
Figures 2 to 5 show the results of the experiments conducted;
Figure 6 shows the comparison between the averages of the scores obtained in the "Vaginal Health Index" by the different groups of patients.

### Detailed description of the invention

According to a first object of the invention, the described vaginal topical composition comprises a mixture isolated from colostrum, rich in biological factors, and ozonized vegetable oils.

In particular, these biological factors include: cytokines, growth factors, chemotactic factors, stem cell stimulating factors, complement proteins, antibacterial and antiviral factors and immunoglobulins.

More specifically, such a mixture isolated from colostrum comprises:
- *Lactoferrin and transferrin.*
   They are involved in the regulation of iron metabolism and concentration, with direct antimicrobial activity, exerted against a wide range of bacteria and viruses.
- *Cytokines*
   They are small soluble proteins that act in an autocrine/paracrine manner by binding to specific cell receptors, operating in networks and orchestrating the development and function of the immune system. Cytokines act at picomolar concentrations. Experiments show that bovine colostrum can modulate the activity of human immune cells *in vitro.* Cytokines, both those linked to innate immunity, such as TNF, IL-1, MCP-1 and IL-15, and those related to the regulation of acquired immunity, such as IL-2, IL-4, IFN-γ, IL-9, and IL-17, have been highlighted as important components of the mixture isolated from colostrum. Among the identified cytokines, IL-17, which is the distinctive cytokine for T helper 17 cells (Th17), is present in high concentrations in the mixture. Interleukin 17 has a fundamental role against bacterial and fungal infections and has a significant pro-inflammatory activity. Recently it has been shown that IL-17 has a regulatory role in hematopoiesis, being involved in the modulation of both hematopoietic and mesenchymal stem cells. High levels of IL-15 were also measured, which induces the proliferation of T, B and natural killer (NK) cells, stimulating the maturation of the immune system and also possesses a unique antitumor activity. It is interesting to note that both proinflammatory stimulation (IL-1, TNF, IFN-γ and IL-12) and anti-inflammatory inhibitory cytokines (IL-10, IL-1RA and TGF-β) are present in the mixture of biological factors isolated from colostrum. Therefore, the use of this balanced product, both *in vitro* and *in vivo,* should ensure avoiding excessive stimulation/inhibition with regard to the delicate regulation of immune responses.
- *Granulocyte colony-stimulating* factor(G-CSF) and *granulocyte-macrophage colony stimulating factor* (GM-CSF)
   They are key substances for the proliferation, maturation and enhancement of progenitor cells in the bone marrow, and their role as stimulating factors in stem cells is quite clear.
- *Growth factors*
   Including peptides belonging to Fibroblast Growth Factors (FGF), Insulin-like Growth Factors (IGF) and Platelet-Derived Growth Factors (PDGF). Because the physiological role of these trophic factors in colostrum is to regulate cell proliferation and differentiation in the gastrointestinal mucosa of the newborn, it is likely that they play a primary role in the regenerative and repairing properties of colostrum in wound healing.

In a preferred aspect, the colostrum used for the purposes of the present invention is obtained from human beings or from a mammal and, preferably, is obtained from cattle.

In fact, it has been found that colostrum of herbivores contains to a greater extent different growth factors and cytokines.

Bovine colostrum is the richest source of biological factors and therefore the most advantageous.

In particular, it has been proved that Holstein (Friesian) cows produce colostrum with the highest concentration of cytokines, growth factors, chemotactic factors, stem cell stimulating factors, complement proteins, antibacterial/antiviral factors and immunoglobulins.

The colostrum used for the purposes of the present invention is preferably collected in the first hours after birth, preferably within five hours.

In a preferred aspect, the cows are preferably at the second or third birth and the colostrum is preferably collected within the fifth hour of delivery, since during this period there is the greatest concentration of active ingredients.

Advantageously, the method for the preparation of the mixture rich in biological factors from colostrum does not provide steps or operations that could damage biological factors by reducing their activity.

For example, heat sterilization steps which could lead to the loss of large quantities of biological factors in the final product as a result of the degradation caused by high temperatures are not carried out.

Or, the separation of casein, necessary as it can lead to allergic reactions, does not provide a coagulation/precipitation step followed by filtration at low pH values, which can result in the denaturation of many proteins, including several biological factors present in the colostrum.

In fact, for the preparation of the mixture rich in biological factors isolated from the colostrum according to the present invention, extraction processes are used which allow obtaining a mixture of factors in a biologically active form.

Processes which can be used for the preparation of the mixture rich in biological factors for the purposes of the present invention are described for example by P. Sacerdote et al. ("Biological components in a standardized derivative of bovine colostrum" - Journal of Dairy Science, Volume 96, Issue 3, 1 March 2013, pages 1745 - 1754) and in the international patent application WO 2011/064114, which describes a process for obtaining a mixture of biological factors isolated from colostrum based on an initial microfiltration step with a 2-6 micron molecular porosity membrane, a collection step of permeate containing biological principles, an optional permeate sterilizing filtration step and an optional step of removal of the water contained in the permeate.

A similar process of extraction from colostrum is described in patent applications WO 2013/098331, WO 2013/098333 and WO 2017/134559.

Other extraction processes may be used, as long as they meet the above requirements.

In a preferred aspect of the invention, the mixture isolated from colostrum and rich in biological factors is obtained according to the process described by P. Sacerdote et al. , which includes the steps of:
1) dilution;
2) skimming;
3) ultrafiltration;
4) dialysis;
5) microfiltration.

With regard to step 1), an amount of 100 kg of colostrum is placed in a reactor and diluted 1:10 with deionized water added with NaCl until reaching the concentration of 0.9%.

In step 2) of skimming, the whole mass is centrifuged at 12.400 g at a temperature of 20-25 °C to remove the fatty part.

In step 3), the previously obtained liquid phase is subjected to ultrafiltration through a membrane with a cut-off of 300 KDa, still maintaining the temperature of 20-25 °C, to remove large proteins (typically caseins) and pathogenic microorganisms.

In the dialysis step 4), the mixture isolated from colostrum and rich in biological factors thus obtained is then dialyzed with a 5 KDa membrane to remove any preservatives or residues of drugs present in the initial colostrum.

In the microfiltration step 5), the resulting product is subjected to a series of sterilizing tangential filtration steps with 0.2 um membranes and subsequently frozen.

The product obtained is therefore characterized by a mixture of biological factors, including also the specific immunoglobulins of the species (IgG, IgA and IgM) with a natural specificity against many bacteria and viruses.

As the last step in the preparation process, the mixture is subjected to a freeze-drying step.

In this way, a sterile powder, free of preservatives, anallergic (casein and lactalbumin are responsible for over 95% of allergy to bovine milk) is obtained, with a very high solubility and with the highest possible concentration of active factors.

In a preferred aspect, a qualitative and quantitative control of biological factors is also provided both *in vitro* and *in vivo.*

To evaluate the quality of the processes implemented to obtain a standardized final product, some key factors of colostrum, such as lactoferrin and transferrin, which are present in high concentrations, are used as markers; in addition, a search for a large number of bioactive factors can be performed on the final product using single known ELISA tests.

In a particularly preferred aspect of the invention, the mixture derived from colostrum is characterized by a content of:
- Cytokines: from 45 to 300 pg/mg, preferably from 50 to 250;
- Growth factors: from 600 to 1900 pg/mg, preferably from 650 to 1850;
- Chemotactic factors: from 2 to 20 pg/mg; preferably from 3 to 15;
- Stimulating factors for stem cells: from 100 to 1200 pg/mg, preferably from 130 to 1100;
- Antibacterial/antiviral factors: from 20 to 80 µg/mg, preferably from 22 to 70;
- C3a/C4a complement proteins: from 1 to 5 pg/mg, preferably from 1.20 to 3;
- Immunoglobulins: from 0.3 to 0.9 mg/mg, preferably from 0.35 to 0.80.

In the case in which it is desired to obtain an even more concentrated mixture of active factors isolated from colostrum it is possible to add further steps to the method described above which allow obtaining a product free of immunoglobulins for an amount larger than at least 80% of the total.

In a preferred aspect of the invention, the immunoglobulins are removed for an amount larger than 85%, even more preferably larger than 90% and much more preferably larger than 95%.

The resulting product has a high activity of biological factors, increasing it by more than 60% because the weight of the active ingredients is much higher at the same weight.

According to another embodiment of the invention, when the mixture isolated from colostrum of the compositions of the invention has been treated to remove the immunoglobulins according to what has been described above, the percentage of ozonized vegetable oil can be kept very low (lower than 0.1%)

For the purposes of the present invention, therefore, a purification step may be carried out for the removal of the immunoglobulins.

In particular, this step is carried out on the concentrated product before freezing and freeze-drying, and is characterized by a depletion step of the IgG and IgA, which, in a preferred aspect of the invention, is carried out by affinity chromatography.

The colostrum purification step also comprises an IgM depletion step, for example conducted by tangential filtration.

METHOD: In a preferred aspect, affinity chromatography is used in the method of the present invention by:
- 1. *CaptureSelect^{™} IgG-Fc* (ms) *Affinity Matrix* (*Thermo Fisher Scientific*) or equivalent product. This system has been specifically designed to purify IgG from different species (human, mouse, rat, rabbit, cow, horse and sheep) by means of high affinity bonds with the Fc region of IgG.
- 2. *CaptureSelect*^{™} *Bovine IgA Affinity Matrix* (*Thermo Fisher Scientific*) or equivalent product. This system has been designed specifically for the purification of bovine IgA from whey/colostrum. In *CaptureSelect*^{™} *Bovine IgA Affinity Matrix,* affinity ligands are created using proprietary technology based on single-domain antibody fragments derived from camelids. The ligand is a 13 kDa single domain fragment comprising the 3 complementary determining regions (CDR) that form the antigen binding domain, effectively produced by the yeast *Saccharomyces cerevisiae* in a process free of animal components.

In a preferred aspect of the present invention, the depletion of IgM and aggregates of an immunoglobulin nature takes place by means of a tangential flow filtration step, which uses membranes with a molecular cut-off of about 750 kDa/0.02 µm. IgM depletion is carried out by ultrafiltration as these molecular species are characterized by a much higher molecular weight than the bioactive component. More in detail, the pentamers of immunoglobulins M, which on average have a molecular weight of 800-900 kDa, may be removed by tangential filtration using membranes of appropriate size, while proteins with a lower molecular weight can pass through it without being retained. Different filtration methods as well as a variety of filters were tested and the best results in terms of IgM depletion and aggregates were obtained with a membrane-coupled tangential filtration system with a cut-off of 750 kDa/0.02 um, type ENTEGRIS Savana^{®} PS cartridge filter 0.02 micron or equivalent product.

In a further preferred aspect of the present invention, the aforementioned concentrated sample is subjected to a lyophilization process.

The process was developed in view of an industrial application. All stages of the process are transferable in view of a large-scale purification process.

As for ozonized vegetable oil, it is a product obtained from the treatment of vegetable oils with ozone.

Ozone is a thermodynamically unstable gas and an energizing oxidant (Eo = 2.07 V), often used as a germicide to purify water. Ozone damages the nucleic acids of the bacterial cell. The proteins and lipids of bacterial membranes are also important ozone targets. Because of its reduced stability, gaseous ozone must be used promptly and appropriately as it presents risks of respiratory toxicity. Alternatively, this gas can be incorporated into liquids such as blood, water or oily carriers. Reaction products with oily vehicle are able to conserve therapeutic power for a long time.

Vegetable oils are characterized by a high percentage of unsaturated fatty acids and a low content of saturated fatty acids. In all vegetable oils the composition may vary according to the cultivar, environmental conditions, harvesting and processing.

According to the present invention, the vegetable oil used for the preparation of the described formulation is selected from the group comprising: sunflower oil, olive oil, argan oil, jojoba oil, linseed oil, macadamia oil, or another vegetable oil.

In a preferred aspect, oil, particularly olive or argan, is an extra virgin olive oil.

In a preferred aspect of the invention, sunflower oil is used.

There are different sunflower oils with different composition, such as:
• SO sunflower oil, *Helianthus annuus,*
• SOHO oil with a high oleic acid content from seeds of a variety derived from sunflower,
• SOMO oil with a medium content of oleic acid from seeds of a variety derived from sunflower.

The fatty acid composition of these oils is shown in the following table:

| **fatty acid** | **no. of C atoms: no. of double bonds and ω** | **SO** | **SOHO** | **SOMO** |
|---|---|---|---|---|
| linoleic acid | 18:2ω6 | 61.15 | 9.55 | 32 |
| oleic acid | 18:1ω9 | 26.7 | 82.9 | 57.45 |
| palmitic acid | 16:0 | 6.3 | 3.8 | 4.8 |
| stearic acid | 18:0 | 4.6 | 4.55 | 3.55 |

The fatty acids, when subjected to ozonation, produce monoozonides of oleic acid and monoozonides and diozonides of linoleic acid as determined by infrared spectrophotometry.

The average number of peroxides of ozonized sunflower oil expressed as meq of oxygen/kg of oil is equal to 700.

Toxicity tests have shown that the LD₅₀ of sunflower ozonized oil is high, equal to 16.5 g/kg and 1.6 mL/kg, when administered intra peritoneally and via os, respectively.

The ozonation of vegetable oils, preferably sunflower oil, to be used as a component of the topical vaginal compositions for use in the treatment of vaginitis and vaginosis, object of the present invention, is carried out according to the techniques known in the prior art.

The standardization of the preparation is carried out according to the following parameters:
- Peroxide Index (PI), indicates the amount of peroxide in the ozonized oil. It is defined as the quantity of active oxygen per kilogram of preparation (mmol Kg⁻¹). In a preferred aspect, the PI is between 500 and 800 mmol Kg⁻¹, preferably 650 mmol Kg⁻¹.
- Acidity Index indicates the free fatty acid within the ozonized oil. It is defined as the number of milligrams of potassium hydroxide needed to neutralize the free fatty acid in a milligram of ozonized oil.

In sunflower oil, the value must range between 6 and 8 units.

In the preparation of the present invention, in which the oil is ozonized, it should preferably not exceed 30 units.
- Concentration of aldehydes. It is measured by adding free hydroxylamine to the aldehyde carboxylic group. The results are expressed in mmol g⁻¹ of ozonized oil; the range must be between 0.4 and 0.9 mmol g⁻¹.
- Iodine Index is a measure of the rate of unsaturation of sunflower oil expressed as the number of grams of iodine that reacts with 100 grams of sunflower oil. In sunflower oil, the value ranges from 125 to 135 units.

In the preparation of the present invention, on the other hand, the value ranges from 50 to 90 units.
- Viscosity is a measure of the polymerization by condensation of peroxides that are formed in the process of ozonation of sunflower oil. Values are expressed in mPa.s (centipoise or cP value; Institute of Standards and Technology). To obtain ozonized sunflower oil with a PI of between 500 and 800, the viscosity must be between 100 and 450 mPa.s.

According to a preferred aspect of the present invention, the ozonized oil may be further enhanced and have a number of peroxides> 800 meqO₂/kg.

In this case the other parameters above must be adapted and standardized accordingly.

In the preparation of the invention, the two components may be present in the following quantities:

| | |
|---|---|
| mixture isolated from colostrum and rich in biological factors | 3-10% (w/w) |
| ozonized vegetable oils | 5-75% (w/w) |

In a preferred aspect of the invention, the two components are present in the following quantities:

| | |
|---|---|
| mixture isolated from colostrum and rich in biological factors | 4-6% (w/w) |
| ozonized vegetable oils | 10-50% (w/w) |

For example, the two components may be present in the following quantities:

| | |
|---|---|
| mixture isolated from colostrum and rich in biological factors | 5% (w/w) |
| ozonized vegetable oils | 10% (w/w) |

According to the present invention, the mixture isolated from colostrum rich in biological factors together with ozonized vegetable oils is used for the preparation of a formulation for vaginal use.

In a preferred aspect, such formulations are represented by gels, vaginal suppositories, douching or other forms suitable for vaginal use.

The man skilled in the art will be able to identify the additives, excipients and other components necessary for obtaining such formulations selecting them from the prior art.

In a preferred aspect of the present invention, the formulation described may contain other ingredients in effective concentration.

In particular, one or more of the following components may be included.

### Lactic acid

The exogenous lactic acid, replacing the one normally produced under physiological conditions by lactobacilli by degradation of glucose, helps to make the vaginal pH acidic.

In one aspect of the invention, lactic acid is a 90% preparation and may be present in an amount of about 0.2% (weight/weight).

### Betaine

Betaine or Trimethylglycine (TMG) is a substance of plant origin extracted from sugar beet. From a structural point of view, Trimethylglycine differs from Dimethylglycine (vitamin B15) due to the presence of a third methyl group.

Trimethylglycine intervenes in trans-methylation, a biochemical process indispensable for cell metabolism and regeneration, by which the methyl groups (CH3) are transferred from one molecule to another.

In one aspect of the invention, betaine may be present in an amount of about 4% (weight/weight).

### Panthenol

Panthenol is the reduced alcoholic form of pantothenic acid (vitamin B5). In various formulations, the use of panthenol is preferred over that of pantothenic acid, due to the greater stability of the alcoholic form. Panthenol has chemical-physical characteristics that make it easy to use, such as water solubility.

In one aspect of the invention, panthenol may be present in an amount of about 0.2% (weight/weight).

### Sericin Integra (MW 400,000)

It is moisturizing, filming and has acidity buffering capacity, and it reduces the aggressiveness of surfactants.

In one aspect of the invention, sericin may be present in an amount of about 4% (weight/weight).

### Vitamin E (or Tocopherol)

The biological action of vitamin E is mainly due to its antioxidant properties: it prevents the oxidation of polyunsaturated fatty acids (PUFA), sequestering the peroxylipid radicals. This seems to be the fundamental function of vitamin E in animal tissues and in particular in cell membranes, where tocopherol is associated with PUFAs in phospholipids. Vitamin E also enters the processes of cellular respiration, fortifies the walls of blood vessels and has important positive effects on the reproductive system.

In one aspect of the invention, vitamin E may be present in an amount of about 0.5% (weight/weight).

### Plant extracts

They can be selected from the extracts of aloe, chamomile, calendula, etc... or other plant substances with positive effects on vaginal health.

These may be added in the form of dry extracts, essential oils or other preparations.

In one aspect of the invention, the plant extract may be present in an amount of about 0.5-1% (weight/weight).

For example, chamomile essential oil may be added in a quantity of 1% (weight/weight), while aloe vera in the form of gel (10:1) may be added in an amount of 0.5% (w/w).

### Hyaluronic acid

Hyaluronic acid is a polysaccharide consisting of non-branched chain glycosaminoglycans widely present in the extracellular matrix of tissues able to bind large quantities of water, helping to keep the epithelium turgid and elastic. The contribution of exogenous hyaluronic acid, for its ability to restore the tone and elasticity of tissues, as well as to improve and accelerate the re-epithelialization process, represents a non-pharmacological approach to the treatment of vaginal diseases and in particular of vaginal atrophy.

In one aspect of the invention, hyaluronic acid may be present in an amount of about 0.2% (weight/weight).

### Metals

They may be selected from the group comprising: silver (colloidal silver, metallic silver or in another therapeutically acceptable form), zinc (zinc sulfate or in another therapeutically acceptable form).

For example, metallic silver (99.9% pure) or zinc sulfate may be present, alone or in combination, in an amount of about 0.05% (weight/weight) each.

In a preferred aspect of the invention, the described formulation comprises:

| Component | % (weight/weight) |
|---|---|
| Mixture isolated from colostrum | 5.00 |
| Ozonized vegetable oil | 10.00 |
| Other components according to the present invention | 9.90 |
| Additives, excipients and preservatives as needed | to 100 |

According to an object of the present invention, the described compositions are advantageously for use in medicine.

According to an aspect of the present invention, the compositions are described for use in the treatment of vaginal dryness, vaginitis and vaginosis.

In a preferred aspect, the compositions are described for use in the treatment of premenopausal, menopausal, hysterectomised, adolescent, nursing or breastfeeding women or women suffering from stress.

Furthermore, the use of the compositions of the invention may follow previous incorrect or unbalanced therapeutic treatments, as happens in the case of drug abuse or the use of inappropriate drugs.

According to an aspect of the invention, the formulations described are for use in a method for the treatment of vaginal dryness, vaginitis and vaginosis.

In particular, this method may relate to premenopausal, menopausal, hysterectomized, adolescent, nursing or breastfeeding women or women suffering from stress.

Furthermore, such a method may follow previous incorrect or unbalanced therapeutic treatments, as happens in the case of drug abuse or the use of inappropriate drugs.

The method comprises the administration (application) to a subject (woman) in need thereof of one of the compositions in a quantity, for a time and a sufficient number of times according to necessity.

The invention is hereinafter described in greater detail by the following experimental part.

### EXAMPLE 1

### Formulation

| Component | % (weight/weight) |
|---|---|
| Mixture isolated from colostrum | 5.00 |
| Ozonized sunflower oil | 10.00 |
| 90% lactic acid | 0.20 |
| Betaine | 4.00 |
| Panthenol | 0.20 |
| Whole sericin (400 Kd) | 4.00 |
| Vitamin E | 0.50 |
| Chamomile essential oil | 1.00 |
| Additives, excipients and preservatives as needed | to 100 |

The composition may possibly be modified by adding the following components, consequently reducing the amount of additives, excipients and preservatives:

| Component | % (weight/weight) |
|---|---|
| Aloe vera gel 10:1 | 0.50 |
| Hyaluronic acid | 0.20 |
| Pure metallic silver (99.9%) | 0.05 |
| Zinc sulfate | 0.05 |

### EXAMPLE 2

### Clinical trials

A total of 60 women of child-bearing, pre-menopausal and menopause age for at least one year, suffering from vaginosis due to infection, hormonal, mechanical reasons, stress, unbalanced local treatments, breastfeeding, contraceptives, etc. were divided into four homogeneous groups of 15 patients each. Of the four groups, group 1) was treated with the composition of Example 1 in the form of vaginal gel, group 2) was treated with the same vaginal gel containing only the derivative of colostrum as the active ingredient, group 3) was treated with the same vaginal gel containing only the ozonized vegetable oil as the active ingredient and group 4) was treated with the same vaginal gel but containing only the derivative of colostrum without immunoglobulins (IgG, IgA, IgM ) for an amount of at least 80% of the total, preferably higher than 85%, even more preferably higher than 90% and much more preferably higher than 95%.

For all four formulations the therapy consisted of a single daily administration for 20 consecutive days.

The pathological conditions of the vagina, pre- and posttreatment, were evaluated using the Vaginal Health Index (Bachman et al., Clin. Pr. Sex., 1992).

The Vaginal Health Index is a validated tool that can be used to assess the level of vaginal atrophy resulting from vaginitis and vaginosis from various causes. It provides for the use of 5 parameters (vaginal elasticity, vaginal secretions, pH, vaginal mucosa integrity and vaginal hydration) to which to assign individual scores whose sum defines the vaginal health condition. If the score is <15, the vagina is considered atrophic.

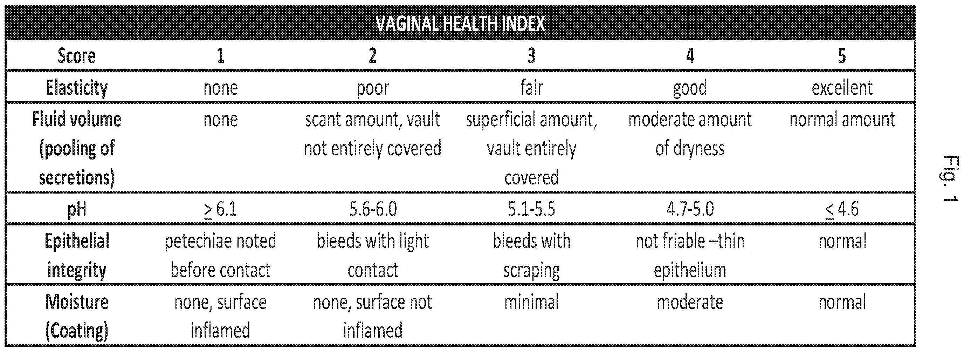

Figure 1 shows the scores to be assigned to each parameter (or index) on the basis of the clinical examination.

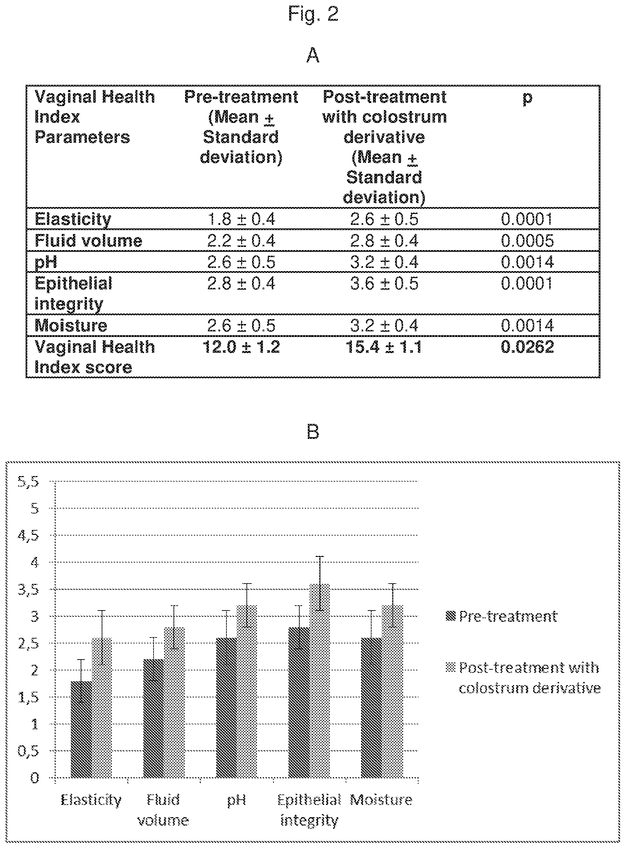

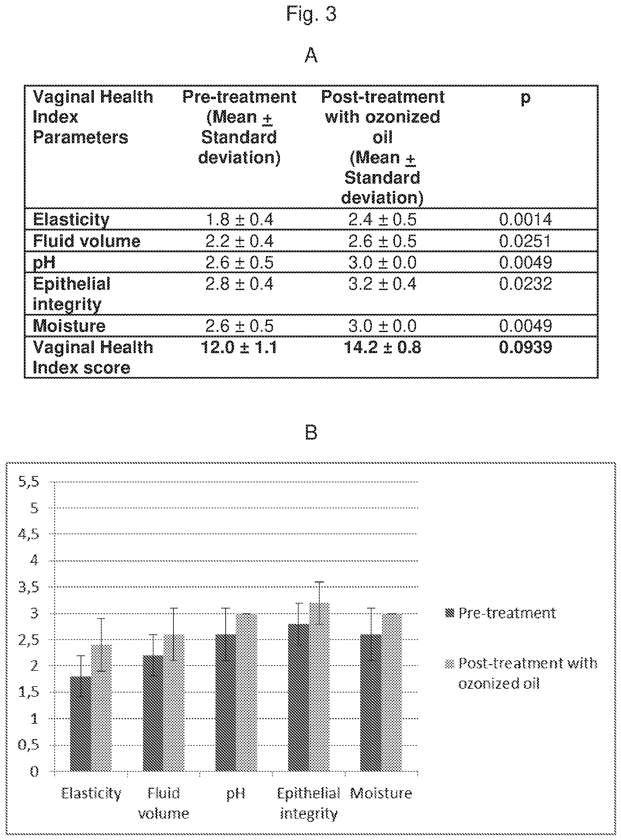

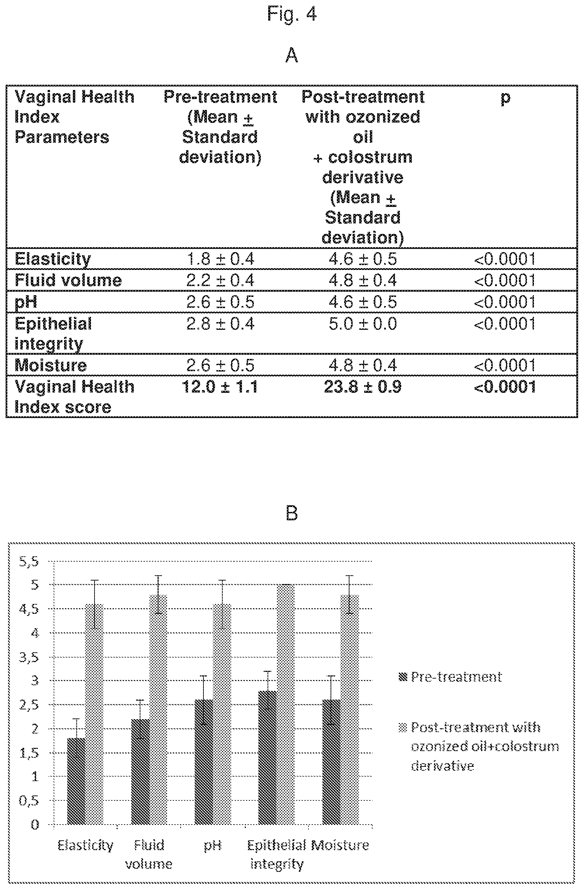

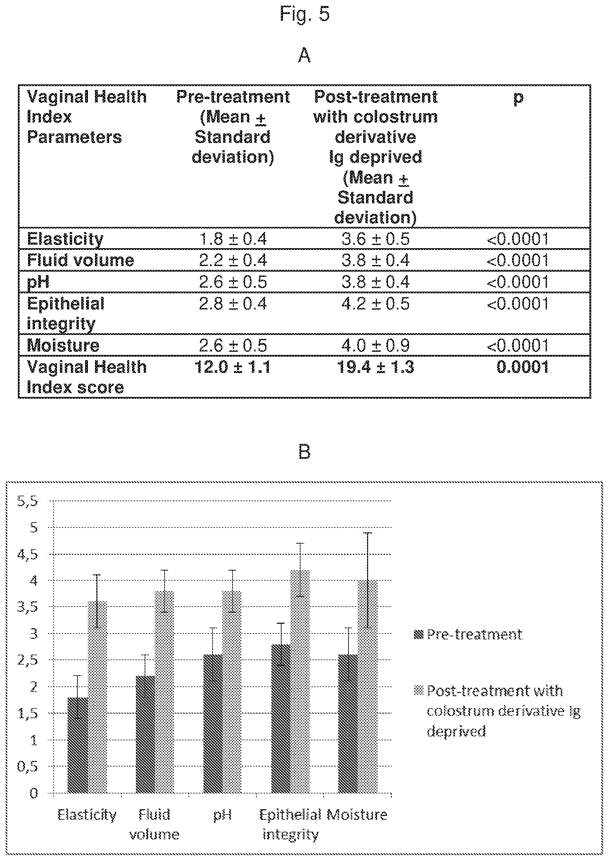

The tables in Figures 2A, 3A, 4A and 5A show the averages of the scores assigned to the 5 parameters that define the *Vaginal Health Index,* before and after the relative treatment with one of the four tested products (derivative of colostrum alone (group 2) ozonized vegetable oil alone (group 3), derivative of colostrum + ozonized vegetable oil (group 1) and derivative of colostrum but without immunoglobulins (group 4)). The same data are shown graphically in Figures 2B, 3B, 4B and 5B respectively. In order to compare the data obtained from the analyses performed, the Student test was used (p <0.005) .

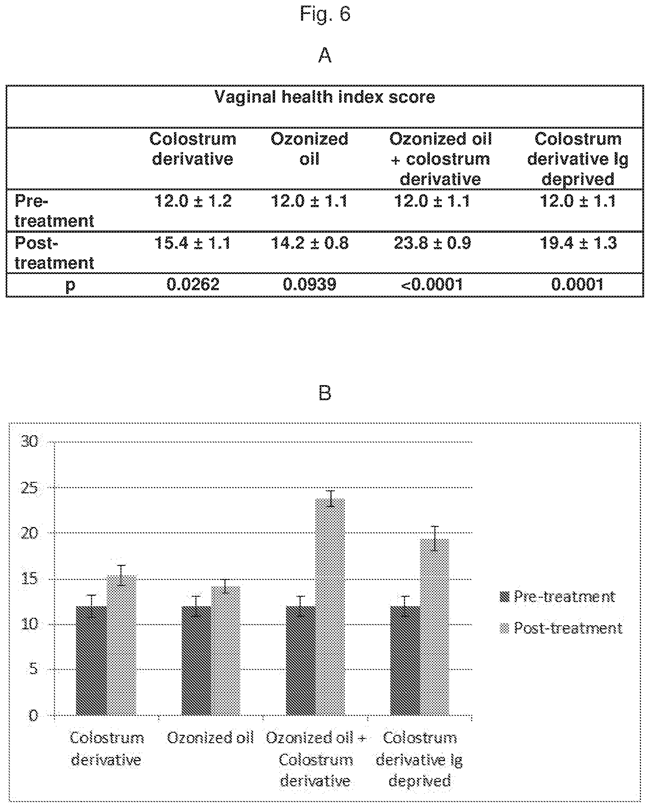

The resulting data showed that the product containing the derivative of colostrum + ozonized vegetable oil, object of the present invention and administered to group 1), obtained from the clinical point of view the greater benefits compared to the other products, as shown by the comparison between the mean scores obtained in the Vaginal Health Index from the different patient groups (figures 6A and 6B). Only the derivative of colostrum without immunoglobulins (IgG, IgA, IgM) (administered in group 4) obtained results comparable to those obtained from the derivative of colostrum + ozonized vegetable oil (of the present invention, administered to group 1).

Statistical calculations show that the difference between the Vaginal Health Index is significant between the derivative of colostrum + ozonized vegetable oil and other reference products, and between the derivative of colostrum without immunoglobulins and other reference products, but also between the derivative of colostrum + ozonized vegetable oil and the derivative of colostrum without immunoglobulins.

The compound object of the present invention therefore allowed restoring a healthy vaginal environment, normalizing the pH, regenerating the vaginal epithelium and increasing the natural lubrication of the vagina, as well as relieving the symptoms related to vaginitis and vaginosis, such as dryness and pain during sexual intercourse or urination. Moreover, the local treatment with the product object of the present invention did not give rise to any collateral effect or endometrial stimulation, thus proving to be not only effective but also safe.

While the two reference compounds (derivative of colostrum alone and ozonized vegetable oil alone) obtained results far lower than the object of the present invention, the derivative of colostrum deprived of immunoglobulins (IgG, IgA, IgM) obtained good results, in some cases very close to those obtained from the derivative of colostrum + ozonized vegetable oil. This is explained by the fact that by eliminating the Ig, which alone make up more than 60% of the derivative of colostrum, the bioactive factors contained therein (growth factors and antibacterial substances such as lysozyme and lactoferrin) are concentrated enormously compensating in part the action of the ozonized vegetable oil.

From the above description of the present invention, the advantages provided by the proposed formulations are immediately apparent. In particular, it was observed that the effectiveness of these formulations is closely linked to the synergy of the two components and their functional interdependence.

The bioactive substances extracted from colostrum in fact, and in this case above all the growth factors, act by promoting the reconstruction and maintenance of the functional integrity of the vaginal mucosa (atrophic or damaged by bacterial and viral agents, detergents, soaps, panty liners, lubricants, non-breathable garments, pharmacological treatments, etc.). The vaginal epithelium thus regenerated can resume its physiological function, the normal protective and lubricating secretions are restored and the whole vaginal ecosystem is restored to an optimal condition.

The ozonized vegetable oil, in full synergy with the antibacterial, antiviral and immunostimulant substances derived from colostrum, acts as a powerful natural antibacterial agent, possessing remarkable inactivating properties against viruses, fungi and protozoal forms, and even better, due to its "natural preservative" function demonstrated by the numerous applications in the food field, it protects the delicate bioactive substances isolated from colostrum.

The clinical tests performed showed a substantially higher activity of the compositions described herein with respect to a product comprising only the portion derived from colostrum or only the portion based on ozonized oil.

Further experimental tests have also shown that the derivative of colostrum deprived of Immunoglobulins (IgG, IgA, IgM) for an amount of at least 80% of the total, preferably higher than 85%, even more preferably higher than 90% and much more preferably higher than 95%, obtained good results, in some cases very close to those obtained from the derivative of colostrum + ozonized vegetable oil. This is explained by the fact that by eliminating the Ig, which alone make up more than 60% of the derivative of colostrum, the bioactive factors contained therein (growth factors and antibacterial substances such as lysozyme and lactoferrin) are concentrated enormously compensating in part the action of the ozonized vegetable oil. The same data are shown graphically in figures 6A and 6B.

A man skilled in the art may make adjustments, changes and replacements with other similar components to the present invention described above in order to meet specific incidental needs, without departing from the scope of the following claims. For example, one or more excipients may be modified according to the selected pharmaceutical form.

For example, one or more excipients may be modified according to the selected pharmaceutical form.

## Claims

1. A vaginal topical composition comprising a mixture isolated from colostrum, rich in biological factors and ozonized vegetable oils.

2. A vaginal topical composition according to the preceding claim, comprising said mixture isolated from colostrum rich in biological factors in an amount of about 3-10% and preferably about 4-6% (weight/weight).

3. A vaginal topical composition according to claim 1 or 2, comprising ozonized vegetable oils in an amount of about 5-75% and preferably about 10-50% (weight/weight).

4. A vaginal topical composition according to any one of the preceding claims, wherein said mixture isolated from colostrum rich in biological factors is isolated from mammalian, human or animal colostrum, preferably animal and more preferably bovine.

5. A vaginal topical composition according to any one of the preceding claims, wherein said mixture isolated from colostrum rich in biological factors is isolated from the colostrum of cows of the Holstein (Friesian) breed.

6. A vaginal topical composition according to any one of the preceding claims, wherein said mixture isolated from colostrum rich in biological factors is isolated from the colostrum collected within the fifth hour of delivery at the second or third delivery.

7. A vaginal topical composition according to any one of the preceding claims, wherein said mixture isolated from colostrum rich in biological factors comprises cytokines, growth factors, chemotactic factors, stem cell stimulating factors, complement proteins, antibacterial and antiviral factors, immunoglobulins.

8. A vaginal topical composition according to any one of the preceding claims, wherein said mixture isolated from colostrum rich in biological factors is free of IgG, IgA, IgM immunoglobulins, at least for 80% of the total, preferably higher than 85%, even more preferably higher than 90% and much more preferably higher than 95%.

9. A vaginal topical composition according to any one of the preceding claims, wherein said mixture isolated from colostrum rich in biological factors is free of IgG, IgA, IgM immunoglobulins at least for 80% of the total, preferably higher than 85%, even more preferably higher than 90% and much more preferably higher than 95%, and does not comprise ozonized vegetable oil.

10. A vaginal topical composition according to any one of the preceding claims, wherein said ozonized vegetable oils are sunflower vegetable oils.

11. A vaginal topical composition according to any one of the preceding claims, wherein said ozonized vegetable oils are represented by a mixture comprising monoozonides of oleic acid, monoozonides and diozonides of linoleic acid.

12. A vaginal topical composition according to any one of the preceding claims, further comprising one or more of the following compounds: lactic acid; moisturizers; emollients; lubricants, for example selected from the group comprising: betaine, panthenol, sericin; vitamins, preferably vitamin E; plant extracts; hyaluronic acid; metals.

13. A vaginal topical composition according to any one of the preceding claims in a suitable formulation selected from the group comprising: gels, vaginal suppositories, douching or other suitable formulations.

14. A vaginal topical composition according to any one of the preceding claims having the following composition:
| Component | % (weight/weight) |
|---|---|
| Mixture isolated from colostrum | 5.00 |
| Ozonized sunflower oil | 10.00 |
| 90% lactic acid | 0.20 |
| Betaine | 4.00 |
| Panthenol | 0.20 |
| Whole sericin (400 Kd) | 4.00 |
| Vitamin E | 0.50 |
| Chamomile essential oil | 1.00 |
| Additives, excipients and preservatives as needed | to 100 |

15. A vaginal topical composition according to the preceding claim, further comprising:
| Component | % (weight/weight) |
|---|---|
| Aloe vera gel 10:1 | 0.50 |
| Hyaluronic acid | 0.20 |
| Pure metallic silver (99.9%) | 0.05 |
| Zinc sulfate | 0.05 |

16. A vaginal topical composition according to any one of the preceding claims for use in medicine.

17. A vaginal topical composition according to the preceding claim for use in the treatment of vaginal dryness.

18. A vaginal topical composition according to claim 16 or 17 for use in the treatment of vaginitis and vaginosis.

19. A vaginal topical composition according to any one of the preceding claims 16 to 18 for use in premenopausal, menopausal, hysterectomized, adolescent, nursing or breastfeeding women, women suffering from stress.

20. A vaginal topical composition for use according to any one of the preceding claims from 16 to 18 for women following prior therapeutic treatments.

## Patentansprüche

1. Vaginale topische Zusammensetzung, umfassend eine aus Kolostrum isolierte Mischung, die reich an biologischen Faktoren ist, und ozonisierte pflanzliche Öle.

2. Vaginale topische Zusammensetzung nach dem vorhergehenden Anspruch, umfassend die aus Kolostrum isolierte Mischung, die reich an biologischen Faktoren ist, in einer Menge von etwa 3-10% und bevorzugt etwa 4-6% (Gewicht/Gewicht).

3. Vaginale topische Zusammensetzung nach Anspruch 1 oder 2, umfassend ozonisierte pflanzliche Öle in einer Menge von etwa 5-75% und bevorzugt etwa 10-50% (Gewicht/Gewicht).

4. Vaginale topische Zusammensetzung nach einem der vorhergehenden Ansprüche, wobei die aus Kolostrum isolierte Mischung, die reich an biologischen Faktoren ist, aus Säugetier-, menschlichem oder tierischem Kolostrum, bevorzugt tierischem und stärker bevorzugt aus Rinder-Kolostrum, isoliert ist.

5. Vaginale topische Zusammensetzung nach einem der vorhergehenden Ansprüche, wobei die aus Kolostrum isolierte Mischung, die reich an biologischen Faktoren ist, aus dem Kolostrum von Kühen der Rasse Holstein (Friesisch) isoliert ist.

6. Vaginale topische Zusammensetzung nach einem der vorhergehenden Ansprüche, wobei die aus Kolostrum isolierte Mischung, die reich an biologischen Faktoren ist, aus dem Kolostrum isoliert ist, das innerhalb der fünften Stunde nach der Entbindung bei der zweiten oder dritten Entbindung gesammelt ist.

7. Vaginale topische Zusammensetzung nach einem der vorhergehenden Ansprüche, wobei die aus Kolostrum isolierte Mischung, die reich an biologischen Faktoren ist, Zytokine, Wachstumsfaktoren, chemotaktische Faktoren, stammzellstimulierende Faktoren, Komplementproteine, antibakterielle und antivirale Faktoren, Immunglobuline umfasst.

8. Vaginale topische Zusammensetzung nach einem der vorhergehenden Ansprüche, wobei die aus Kolostrum isolierte Mischung, die reich an biologischen Faktoren ist, frei von IgG-, IgA-, IgM-lmmunglobulinen, zumindest für 80% der Gesamtmenge, bevorzugt mehr als 85%, noch stärker bevorzugt mehr als 90% und noch viel stärker bevorzugt mehr als 95%, ist.

9. Vaginale topische Zusammensetzung nach einem der vorhergehenden Ansprüche, wobei die aus Kolostrum isolierte Mischung, die reich an biologischen Faktoren ist, frei von IgG-, IgA-, IgM-lmmunglobulinen, zumindest für 80% der Gesamtmenge, bevorzugt mehr als 85%, noch stärker bevorzugt mehr als 90% und noch viel stärker bevorzugt mehr als 95%, ist und kein ozonisiertes pflanzliches Öl umfasst.

10. Vaginale topische Zusammensetzung nach einem der vorhergehenden Ansprüche, wobei die ozonisierten pflanzlichen Öle pflanzliche Sonnenblumenöle sind.

11. Vaginale topische Zusammensetzung nach einem der vorhergehenden Ansprüche, wobei die ozonisierten pflanzlichen Öle durch eine Mischung dargestellt sind, die Monoozonide von Ölsäure, Monoozonide und Diozonide von Linolsäure umfasst.

12. Vaginale topische Zusammensetzung nach einem der vorhergehenden Ansprüche, ferner umfassend eine oder mehrere der folgenden Verbindungen: Milchsäure; Feuchthaltemittel; Linderungsmittel; Gleitmittel, beispielsweise ausgewählt aus der Gruppe umfassend: Betain, Panthenol, Sericin; Vitamine, bevorzugt Vitamin E; Pflanzenextrakte; Hyaluronsäure; Metalle.

13. Vaginale topische Zusammensetzung nach einem der vorhergehenden Ansprüche in einer geeigneten Formulierung, ausgewählt aus der Gruppe umfassend: Gele, Vaginalzäpfchen, Spülung oder andere geeignete Formulierungen.

14. Vaginale topische Zusammensetzung nach einem der vorhergehenden Ansprüche mit der folgenden Zusammensetzung:
| Komponente | % (Gewicht/Gewicht) |
|---|---|
| Aus Kolostrum isolierte Mischung | 5,00 |
| Ozonisiertes Sonnenblumenöl | 10,00 |
| 90% Milchsäure | 0,20 |
| Betain | 4,00 |
| Panthenol | 0,20 |
| Sericin Integra (400 Kd) | 4,00 |
| Vitamin E | 0,50 |
| Ätherisches Öl der Kamille | 1,00 |
| Zusatzstoffe, Hilfsstoffe und Konservierungsstoffe nach Bedarf | bis zu 100 |

15. Vaginale topische Zusammensetzung nach dem vorhergehenden Anspruch, ferner umfassend:
| Komponente | % (Gewicht/Gewicht) |
|---|---|
| Aloe Vera-Gel 10:1 | 0,50 |
| Hyaluronsäure | 0,20 |
| Reines metallisches Silber (99,9%) | 0,05 |
| Zinksulfat | 0,05 |

16. Vaginale topische Zusammensetzung nach einem der vorhergehenden Ansprüche zur Verwendung in der Medizin.

17. Vaginale topische Zusammensetzung nach dem vorhergehenden Anspruch zur Verwendung bei der Behandlung von vaginaler Trockenheit.

18. Vaginale topische Zusammensetzung nach Anspruch 16 oder 17 zur Verwendung bei der Behandlung von Vaginitis und Vaginose.

19. Vaginale topische Zusammensetzung nach einem der vorhergehenden Ansprüche 16 bis 18 zur Verwendung in prämenopausalen, menopausalen, hysterektomierten, jugendlichen, säugenden oder stillende Frauen, Frauen, die unter Stress leiden.

20. Vaginale topische Zusammensetzung zur Verwendung nach einem der vorhergehenden Ansprüche 16 bis 18 für Frauen nach vorangegangenen therapeutischen Behandlungen.

## Revendications

1. Composition topique vaginale comprenant un mélange isolé à partir de colostrum riche en facteurs biologiques et d'huiles végétales ozonisées.

2. Composition topique vaginale selon la revendication précédente, comprenant ledit mélange isolé à partir de colostrum riche en facteurs biologiques en une quantité d'environ 3 à 10 %, et de préférence d'environ 4 à 6 % (en poids).

3. Composition topique vaginale selon la revendication 1 ou 2, comprenant des huiles végétales ozonisées en une quantité d'environ 5 à 75 %, et de préférence d'environ 10 à 50 % (en poids).

4. Composition topique vaginale selon l'une quelconque des revendications précédentes, dans laquelle ledit mélange isolé à partir de colostrum riche en facteurs biologiques est isolé à partir de colostrum de mammifère, à partir de colostrum humain ou animal, de préférence à partir de colostrum animal, et de façon plus préférée à partir de colostrum bovin.

5. Composition topique vaginale selon l'une quelconque des revendications précédentes, dans laquelle ledit mélange isolé à partir de colostrum riche en facteurs biologiques est isolé à partir de colostrum de vaches de race Holstein (frisonne).

6. Composition topique vaginale selon l'une quelconque des revendications précédentes, dans laquelle ledit mélange isolé à partir de colostrum riche en facteurs biologiques est isolé à partir de colostrum collecté dans la cinquième heure suite au vêlage lors de la deuxième ou de la troisième mise bas.

7. Composition topique vaginale selon l'une quelconque des revendications précédentes, dans laquelle ledit mélange isolé à partir de colostrum riche en facteurs biologiques comprend des cytokines, des facteurs de croissance, des facteurs chémotactiques, des facteurs de stimulation de cellules souches, des protéines du complément, des facteurs antibactériens et antiviraux, des immunoglobines.

8. Composition topique vaginale selon l'une quelconque des revendications précédentes, dans laquelle ledit mélange isolé à partir de colostrum riche en facteurs biologiques est dépourvu d'immunoglobines G (IgG), A (IgA), M (IgM), au moins pour 80 % du total, de préférence pour plus de 85 %, de façon plus préférée pour plus de 90 % et de façon encore plus préférée pour plus de 95 %.

9. Composition topique vaginale selon l'une quelconque des revendications précédentes, dans laquelle ledit mélange isolé à partir de colostrum riche en facteurs biologiques est dépourvu d'IgG, d'IgA, d'IgM au moins pour 80 % du total, de préférence pour plus de 85 %, de façon plus préférée pour plus de 90 % et de façon encore plus préférée pour plus de 95 %, et ne comprend pas d'huile végétale ozonisée.

10. Composition topique vaginale selon l'une quelconque des revendications précédentes, dans laquelle lesdites huiles végétales ozonisées sont des huiles végétales de tournesol.

11. Composition topique vaginale selon l'une quelconque des revendications précédentes, dans laquelle lesdites huiles végétales ozonisées sont représentées par un mélange comprenant des monoozonides d'acide oléique, des monoozonides et des diozonides d'acide linoléique.

12. Composition topique vaginale selon l'une quelconque des revendications précédentes, comprenant en outre un ou plusieurs des composés suivants : de l'acide lactique ; des agents hydratants ; des émollients ; des lubrifiants, par exemple choisis dans le groupe comprenant : de la bétaïne, du panthénol, de la séricine ; des vitamines, de préférence de la vitamine E ; des extraits de plante ; de l'acide hyaluronique ; des métaux.

13. Composition topique vaginale selon l'une quelconque des revendications précédentes sous une forme galénique adaptée choisie dans le groupe comprenant : des gels, des suppositoires vaginaux, des produits pour la douche ou d'autres formes galéniques adaptées.

14. Composition topique vaginale selon l'une quelconque des revendications précédentes présentant la composition suivante :
| Composant | % en poids |
|---|---|
| Mélange isolé à partir de colostrum | 5,00 |
| Huile de tournesol ozonisée | 10,00 |
| Acide lactique à 90 % | 0,20 |
| Bétaïne | 4,00 |
| Panthénol | 0,20 |
| Séricine entière (400 Kd) | 4,00 |
| Vitamine E | 0,50 |
| Huile essentielle de camomille | 1,00 |
| Additifs, excipients et agents de conservation si nécessaire | Jusqu'à 100 |

15. Composition topique vaginale selon la revendication précédente, comprenant en outre :
| Composant | % en poids |
|---|---|
| Gel d'aloe vera 10:1 | 0,50 |
| Acide hyaluronique | 0,20 |
| Argent métallique pur (99,9 %) | 0,05 |
| Sulfate de zinc | 0,05 |

16. Composition topique vaginale selon l'une quelconque des revendications précédentes destinée à un usage médical.

17. Composition topique vaginale selon la revendication précédente, destinée à être utilisée dans le traitement de la sécheresse vaginale.

18. Composition topique vaginale selon la revendication 16 ou 17, destinée à être utilisée dans le traitement de la vaginite et de la vaginose.

19. Composition topique vaginale selon l'une quelconque des revendications 16 à 18, destinée à être utilisée chez les femmes en préménopause, les femmes ménopausées, les femmes hystérectomisées, les adolescentes, les femmes allaitantes, les femmes souffrant de stress.

20. Composition topique vaginale destinée à être utilisée selon l'une quelconque des revendications précédentes 16 à 18 chez des femmes suite à des traitements thérapeutiques prescrits antérieurement.
